# EUROPEAN PATENT SPECIFICATION

(11) **EP 3 689 413 B1**
(45) Date of publication and mention of the grant of the patent: **04.10.2023**
(21) Application number: 18858966.7
(22) Date of filing: 01.08.2018
(51) Int. Cl.: A61N 1/05, A61N 1/362, A61B 17/00, A61M 25/01, A61N 1/372, A61B 5/00

(54) **DEVICE FOR PLACING, IN HIS BUNDLE, PACEMAKER LEAD TIP HAVING PASSED THROUGH CORONARY SINUS**
VORRICHTUNG ZUM EINSETZEN VON HERZSCHRITTMACHER-ELEKTRODENSPITZEN DURCH KORONARSINUS IN HIS-BÜNDEL
DISPOSITIF DE MISE EN PLACE, DANS LE FAISCEAU DE HIS, D'UNE POINTE DE SONDE DE STIMULATEUR CARDIAQUE AYANT TRAVERSÉE UN SINUS CORONAIRE

(30) Priority: 25.09.2017 KR 20170123197
(43) Date of publication of application: 05.08.2020
(73) Proprietor: Tau Medical Inc., Busan (KR)
(72) Inventor: KIM, June Hong, Busan 48516 (KR)
(74) Representative: dompatent von Kreisler Selting Werner - Partnerschaft von Patent- und Rechtsanwälten mbB
(86) International application number: PCT/KR2018/008765
(87) International publication number: WO 2019/059518

(56) References cited:
- WO-A1-2016/024710
- JP-A- 2017 502 766
- JP-A- 2017 514 553
- JP-A- 2017 521 117
- KR-A- 20160 011 530
- KR-A- 20160 020 887
- KR-B1- 101 773 207
- US-A1- 2010 317 981
- US-A1- 2011 077 507
- US-A1- 2014 073 978
- US-A1- 2015 196 214
- US-A1- 2015 366 476
- US-A1- 2017 202 469
- US-A1- 2017 209 686

## Description

### Technical Field

The present invention relates to a device for placing, in the His bundle, a pacemaker lead tip having passed through the coronary sinus. More particularly, the present invention relates to a device for placing, in the His bundle, a pacemaker lead tip having passed through the coronary sinus, the device being proposed as part of a more effective method of delivering electrical stimulation in treatments using a pacemaker for patients with cardiac arrhythmia.

### Background Art

Since the first artificial pacemaker was introduced by Furman and Robinson in 1958, a pacemaker has been used as a primary treatment method for patients with bradyarrhythmia. Recently, the pacemaker has been used as a major treatment for arrhythmia diseases such as complete atrioventricular block, high degree atrioventricular block, and sick sinus syndrome. Pacemaker treatment is a method of artificially generating electrical stimulation by the pacemaker because the electrical stimulation of the heart is abnormally conducted.

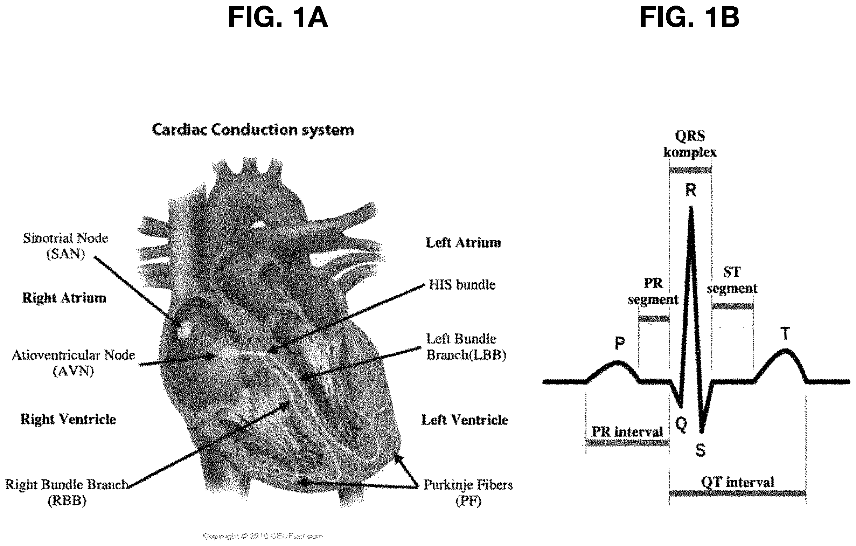

FIG. 1 is a diagram of a cardiac conduction system. Referring to FIG. 1, the cardiac conduction system consists of components conducting signals, starting with the sinoatrial node in the atrium, followed by the atrioventricular node in the atrium and the right bundle and left bundle being branched in the His bundle of the ventricles, and ending with the Purkinje fibers.

In an electrocardiogram, QRS wave is formed by the depolarization process of ventricular muscle, wherein the first down wave following P wave is called Q wave, the first up wave is called R wave, and the down wave following R wave is called S wave. The width of the QRS means the time for conduction of electricity from the His bundle to the entire ventricle. In the normal state, the width of the QRS wave is about 0.12 seconds or less (around 90 ms). In the case of having 0.12 seconds (120 ms) or more, the width of the QRS wave suggests a ventricular conduction disorder. The longer the conduction time, the wider the QRS width. Also, the shorter the conduction time, the narrower the QRS width. A wide QRS represents ventricular desynchronization in which ventricular movements are not unified, causing a side effect resulting in loss of ventricular function.

Therefore, there is a need for research on a safe and easy method as well as an effective method in which the conduction time is shortened and a narrow QRS may be obtained by applying electrical stimulation at a position nearby the conduction system located in the ventricular septum.

In an effort to accomplish this end, the present inventor has proposed and filed patents for a method and a device for placing a pacemaker lead inside the ventricular septum in Korean Patent Application Publication Nos. 10-2016-0020887 and 10-2016-0011530.

US 2017/209686 A1 and US 2017/202469 A1 represent further background art.

### Disclosure

### Technical Problem

The problem to be solved by the present invention is to easily detect the His bundle positioned in the ventricular septum and to apply electrical stimulation to the His bundle, so as to provide fast electrical conduction throughout the entire ventricles.

In addition, the present invention is to provide a device for safely and easily inserting a lead tip of a pacemaker into the His bundle.

The objectives of the present invention are not limited to the above-mentioned objectives, and other objectives that are not mentioned will be clearly understood by those skilled in the art from the following description.

### Technical Solution

In order to achieve the objective of the present invention, there is provided in claim 1 a device for placing, in the His bundle, a pacemaker lead tip having passed through the coronary sinus, the device including: a surgical wire; a capture catheter having an electrocardiogram sensor installed therein to identify a position of the His bundle in the ventricular septum, and capturing the surgical wire positioned in the right ventricle; and a pacemaker lead having a lumen formed therein into which the surgical wire is inserted so that the pacemaker lead tip is inserted into the His bundle along the surgical wire.

The capture catheter may include: a central catheter having a lumen formed therein; an external catheter having the central catheter inserted therein, the central catheter being movable upward and downward; a mesh net having an upper part thereof fixed to the central catheter in a folded state and a lower part thereof fixed to the external catheter in a folded state; and the electrocardiogram sensor coupled to the mesh net and detecting the position of the His bundle so as to verify the position of the His bundle in the ventricular septum.

In the electrocardiogram sensor, a plurality of electrocardiogram sensors may be capable of being coupled to the mesh net, whereby the plurality of electrocardiogram sensors may be coupled to each different position.

The capture catheter may be provided with one or more binders binding the mesh net and the central catheter, and the binders may move upward and downward along the central catheter as the mesh net is unfolded and folded so that the mesh net may have a D shape when being unfolded.

A radiopaque mark may be provided at a position on a surface of the electrocardiogram sensor to be visually identifiable by an imaging device.

The pacemaker lead tip may have a pointed shape to easily penetrate the ventricular septum.

In order to deliver an effective electrical stimulation, the capture catheter may be provided with an electrocardiogram sensor installed therein used to place the pacemaker lead end, which has passed through the coronary sinus, in the His bundle in the ventricular septum where the cardiac conduction system is located, wherein a capture catheter includes an electrocardiogram sensor attached to a side thereof to detect a change in the electrocardiogram to verify a position of the His bundle in the ventricular septum.

The capture catheter may include: a central catheter having a lumen formed therein; an external catheter having the central catheter inserted therein, the central catheter being movable upward and downward; a mesh net having an upper part thereof fixed to the central catheter in a folded state and a lower part thereof fixed to the external catheter in a folded state; and the electrocardiogram sensor coupled to the mesh net and detecting the position of the His bundle so as to verify the position of the His bundle in the ventricular septum.

The capture catheter may be provided with one or more binders binding the mesh net and the central catheter, and the binders may move upward and downward along the central catheter as the mesh net is unfolded and folded so that the mesh net may have a D shape when being unfolded.

In the electrocardiogram sensor, a plurality of electrocardiogram sensors may be capable of being coupled to the mesh net, whereby the plurality of electrocardiogram sensors may be coupled to each different position.

A radiopaque mark may be provided at a position on a surface of the electrocardiogram sensor to be visually identifiable by an imaging device.

### Advantageous Effects

As described above, a device for placing, in the His bundle, a pacemaker lead tip having passed through the coronary sinus according to the present invention may be capable of identifying the position of the His bundle by using a capture catheter coupled to an electrocardiogram sensor.

In addition, since a surgical wire passes through the His bundle and the pacemaker lead is inserted into the His bundle along the surgical wire, electrical stimulation may be directly applied to the His bundle.

### Description of Drawings

FIG. 1 is a diagram of a cardiac conduction system, FIG. 1a is a flowchart of the cardiac conduction system, and FIG. 1b is a diagram showing a waveform of an electrocardiogram.
FIG. 2 is a perspective view of a capture catheter according to a preferred exemplary embodiment of the present invention. FIG. 2a is a perspective view in the case when the capture catheter is folded, FIG 2b is a perspective view in the case when the capture catheter is unfolded, and FIG. 2c is a perspective view in the case when the capture catheter, coupled to a plurality of electrocardiogram sensors, is unfolded.
FIG. 3 is a flowchart of a method for placing a lead tip of a pacemaker having passed through the coronary sinus in the His bundle according to the preferred exemplary embodiment of the present invention.
FIG. 4 is a photograph showing a balloon catheter according to the preferred exemplary embodiment of the present invention.
FIG. 5 is a photograph showing a pressurized septal venogram when the coronary sinus is blocked by using the balloon catheter according to the preferred exemplary embodiment of the present invention.
FIG. 6 is a perspective view showing a safe zone passing catheter according to the preferred exemplary embodiment of the present invention.
FIG. 7 is a perspective view showing a detection of the His bundle with the capture catheter according to the preferred exemplary embodiment of the present invention. FIG. 7a shows a case where one electrocardiogram sensor is provided, and FIG. 7b shows a case where a plurality of electrocardiogram sensors is provided.
FIG. 8 is a diagram showing an electrocardiogram waveform when the electrocardiogram sensor is in contact with a part of the His bundle.
FIG. 9 is a schematic cross-sectional view of a perforating catheter according to the preferred exemplary embodiment of the present invention. FIG. 9a shows a case where the lengths of a first tube and a second tube are the same, and FIG. 9b shows a case where the lengths of the first tube and the second tube are different from each other.
FIG. 10 is a perspective cross-sectional view showing an insertion of a surgical wire, having passed through the His bundle, into a mesh net of the unfolded capture catheter according to the preferred exemplary embodiment of the present invention.
FIG. 11 is a perspective cross-sectional view showing the capture catheter capturing the surgical wire having passed through the His bundle, and guiding the surgical wire to the inferior vena cava, according to the preferred exemplary embodiment of the present invention.

### <Description of the Reference Numerals in the Drawings>

- 10 :: surgical wire
- 20 :: balloon catheter
- 30 :: perforating catheter
- 32 :: first tube
- 34 :: second tube
- 35 :: inclined guide part
- 36 :: supporting wire
- 38 :: second tube radiopaque mark
- 40 :: guidewire
- 50 :: safe zone passing catheter
- 52 :: locking means
- 60 :: capture catheter
- 62 :: central catheter
- 64 :: external catheter
- 66 :: mesh net
- 67 :: binder
- 68 :: electrocardiogram sensor
- 69 :: radiopaque mark
- 70 :: pacemaker lead

### Best Mode

Benefits and features of the present invention and methods of accomplishing the same may be understood more readily by reference to the following detailed description of exemplary embodiments and the accompanying drawings. However, the present disclosure may be embodied in many different forms, and should not be construed as being limited to the exemplary embodiments set forth herein. Rather, these exemplary embodiments are provided so that this disclosure will be thorough and complete and will fully convey the concept of the invention to those skilled in the art, and the present disclosure will only be defined by the appended claims.

Hereinafter, exemplary embodiments of the present invention will be described in detail with reference to the accompanying drawings. Regardless of the drawings, the same reference numbers refer to the same components, and "and / or" includes each and every combination of one or more of the items mentioned.

It will be understood that, although the terms first, second, etc. may be used herein to describe various elements, these elements should not be limited by these terms. These terms are only used to distinguish one element, from another element. Therefore, it is apparent that the first element mentioned hereinbelow may be the second element within the technical scope of the present invention.

The terminology used herein is for the purpose of describing particular embodiments only and is not intended to be limiting of the invention. In this description, the singular also includes the plural unless specifically stated otherwise in the phrase. As used herein, "comprises" and / or "comprising" does not exclude the presence or addition of one or more other components in addition to the mentioned components.

Unless otherwise defined, all terms (including technical and scientific terms) used in the present description may be used in a sense that can be commonly understood by those skilled in the art. In addition, the terms defined in the commonly used dictionaries are not ideally or excessively interpreted unless they are specifically defined clearly.

Hereinafter, the preferred exemplary embodiments of the present invention will be described in detail with reference to the accompanying drawings.

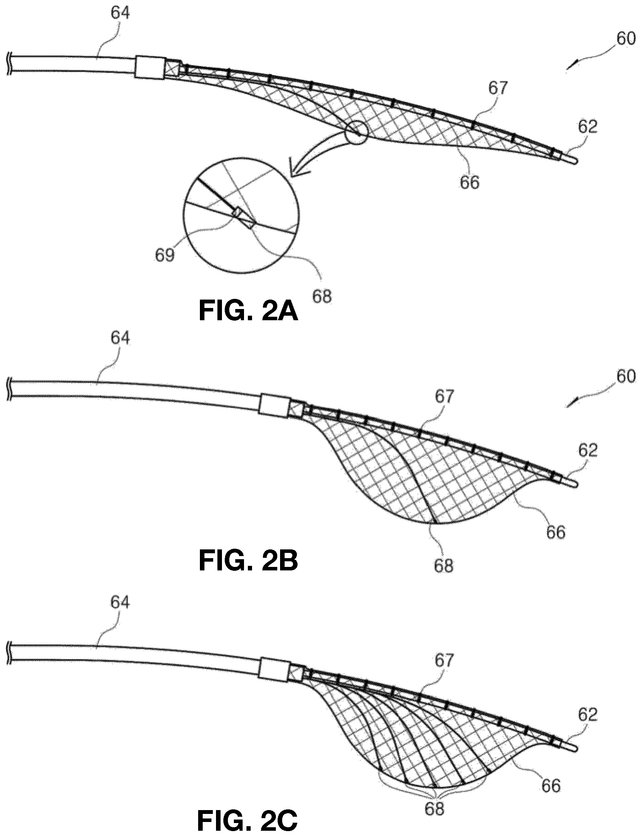

FIG. 2 is a perspective view of a capture catheter according to a preferred exemplary embodiment of the present invention. FIG. 2a is a perspective view in the case when the capture catheter is folded, FIG 2b is a perspective view in the case when the capture catheter is unfolded, and FIG. 2c is a perspective view in the case when the capture catheter, coupled to a plurality of electrocardiogram sensors, is unfolded.

Referring to FIG. 2, the capture catheter 60 according to the preferred exemplary embodiment of the present invention includes a central catheter 62, an external catheter 64, a mesh net 66, and an electrocardiogram sensor 68.

The central catheter 62 has a lumen formed therein into which a guidewire 40 is inserted, and the external catheter 64 has the central catheter 62 inserted thereinto, and is movable upward and downward by the force applied from outside of the patient's body.

The upper part of the mesh net 66 is fixed to the central catheter 62 in a folded state, and the lower part of the mesh net 66 is fixed to the external catheter 64 in a folded state. When the external catheter 64 is pushed upward, the mesh net 66 is unfolded, and when the external catheter 64 is pulled downward, the mesh net 66 is folded.

One or more binders 67 are provided to bind the mesh net 66 and the central catheter 62, wherein the binders 67 move upward and downward along the central catheter 62 as the mesh net 62 is unfolded and folded so that the mesh net 62 has a D shape when being unfolded.

The electrocardiogram sensor 68 is coupled to the mesh net 62 and, more particularly, to a part of the mesh net that protrudes convexly in a D shape when the mesh net 62 is unfolded, and is moved to the ventricular septum to detect the electrocardiogram. The electrocardiogram sensor 68 may be provided with one electrocardiogram sensor, as shown in FIGS. 2a and 2b, or a plurality of electrocardiogram sensors may be coupled to the mesh net, as shown in FIG. 2c. Since the electric wires coupled to the lower part of the electrocardiogram sensor 68 may be coupled to the mesh net 62, unlike those shown in FIG. 2, it is apparent that the electric wires may not be installed inside the mesh net 62. When being coupled, the plurality of electrocardiogram sensors 68 is preferably coupled to each different position on the mesh net. In addition, a radiopaque mark 69 is provided at a position on the upper part of the electrocardiogram sensor 68 so that the position of the electrocardiogram sensor 68 may be visually verified by an imaging device.

Including the capture catheter 60, the device for placing, in the His bundle, the pacemaker lead tip having passed through the coronary sinus, and a method of using the same will be described below.

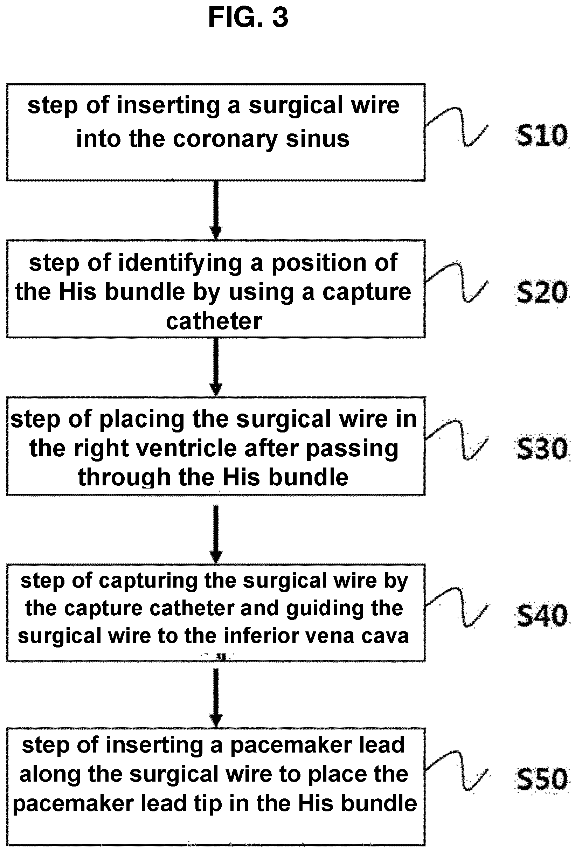

FIG. 3 is a flowchart of a method for placing a lead tip of the pacemaker having passed through the coronary sinus in the His bundle according to the preferred exemplary embodiment of the present invention.

Referring to FIG. 3, first, the step S10 of inserting a surgical wire into the coronary sinus is a step for moving the surgical wire 10 to the superior vena cava, the coronary sinus, and the septal vein in sequence.

The device for placing, in the His bundle, the pacemaker lead tip having passed through the coronary sinus of the present invention includes the surgical wire 10 and the balloon catheter 20. The surgical wire 10 is inserted through the superior vena cava, the coronary sinus, and the septal vein in sequence and passed through the His bundle, and then guided into the inferior vena cava before both sides of the surgical wire are fixed by the operator, so that the surgical wire plays a role of supporting the pacemaker lead 70 to be inserted into the His bundle by inserting through the coronary sinus. Since the current step S10 is a step before detecting the position of the His bundle, the surgical wire 10 is moved to a position at the septal vein and awaits a next move.

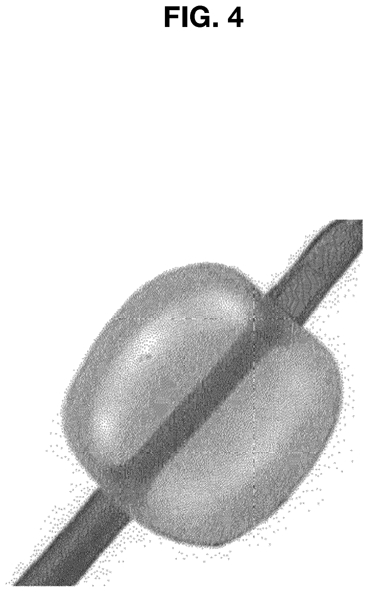

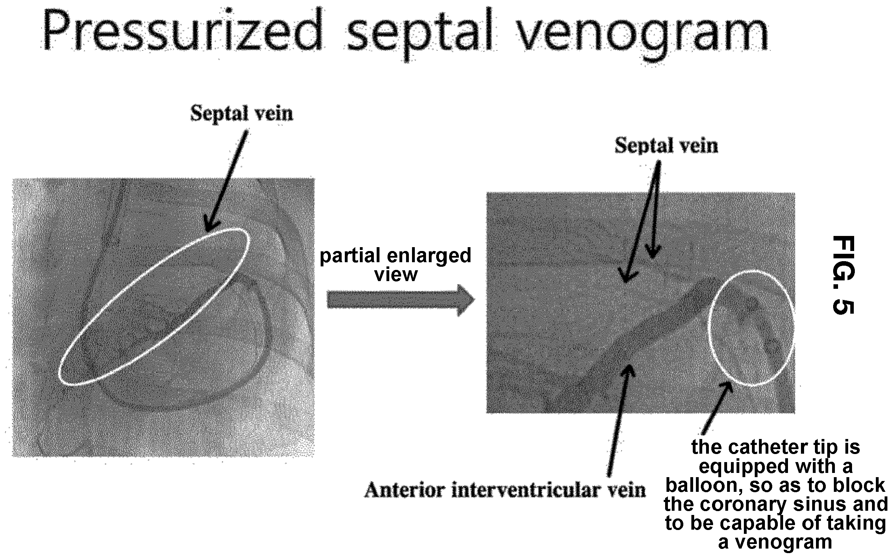

FIG. 5 is a photograph showing a pressurized septal venogram when the coronary sinus is blocked by using the balloon catheter according to the preferred exemplary embodiment of the present invention. The balloon catheter 20 is to facilitate the surgical wire 10 to move to the septal vein. The balloon catheter 20 has a lumen provided therein through which the surgical wire 10 passes as shown in FIG. 4. In general, the septal vein is difficult to identify by the operator, so the balloon catheter 20 is inserted into the coronary sinus, and then the air is injected from the outside to make the balloon to be inflated, thereby blocking the flow of blood in the coronary sinus to cause the coronary sinus to be swollen. Afterward, the pressurized venogram is examined to identify the septal vein positioned in the ventricular septum.

The next step S20 is to identify the position of the His bundle by using the capture catheter. The capture catheter 60 is as described in FIG. 2 and coupled to one or a plurality of electrocardiogram sensors 68. The capture catheter 60 moves to the right ventricle through the inferior vena cava (IVC) and the tricuspid valve, and includes a safe zone passing catheter 50 for the capture catheter 60 to move safely.

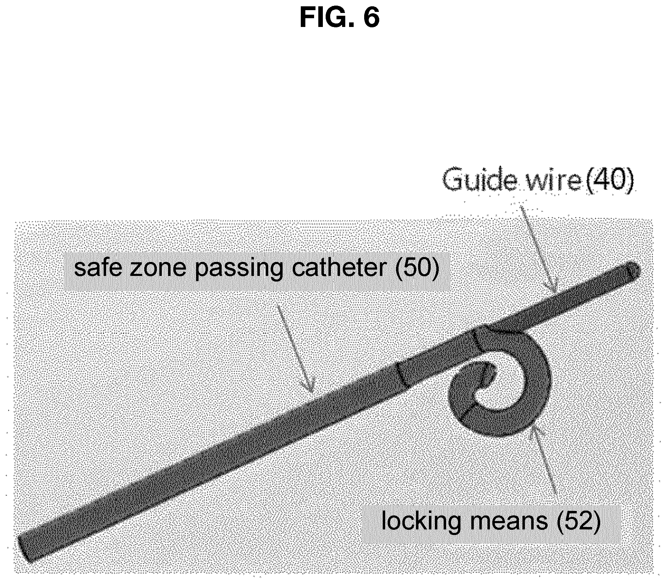

FIG. 6 is a photograph showing a safe zone passing catheter 50 according to the preferred exemplary embodiment of the present invention. Referring to FIG. 6, the safe zone passing catheter 50 has a locking means 52 provided at the upper end thereof, and a lumen provided therein through which the guidewire 40 is inserted. A movement from the inferior vena cava to the tricuspid valve may damage the structures in the heart. By passing through the safe zone other than the unsafe zone where the leaflets of the tricuspid valve, the subvalvular structures such as the chordae tendineae and the papillary muscles, and the modulator band are included, the structures in the heart should be avoided from damaging. The locking means 52 may have a pigtail shape or a balloon-like shape as shown in FIG. 6. The locking means 52 prevents the safe zone passing catheter from moving forward by being blocked by the structure in the heart when passing through the unsafe zone, whereas allowing the safe zone passing catheter to pass freely to the safe zone. Therefore, the locking means may enable the safe zone passing catheter to pass through the safe zone only. After the safe zone passing catheter 50 is moved to the inferior vena cava, the tricuspid valve, and the right ventricle in sequence, the guidewire 40 is inserted into the lumen inside the safe zone passing catheter. When the guidewire 40 is inserted up to the pulmonary artery, the safe zone passing catheter 50 is removed out of the patient's body. The capture catheter 60 is inserted into the patient's body along the guidewire 40 already inserted, and at this time, the capture catheter 60 is inserted while being kept folded. When the capture catheter 60 is positioned in the right ventricle, the external catheter 64 is pushed to unfold the mesh net 66 in the D shape, and the position of the His bundle is detected by the electrocardiogram sensor 68.

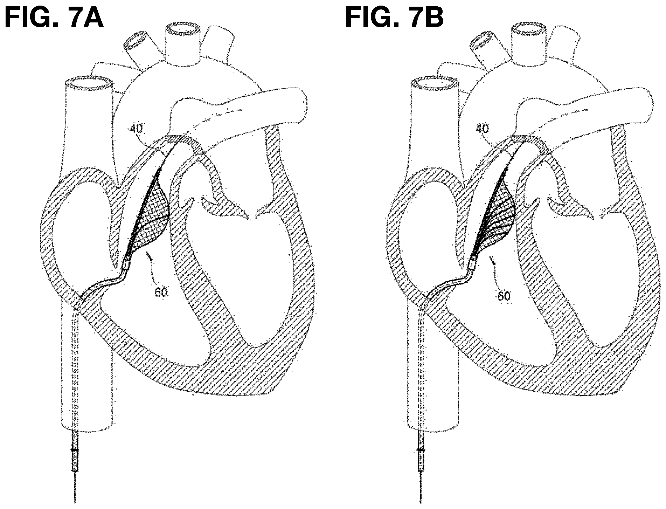

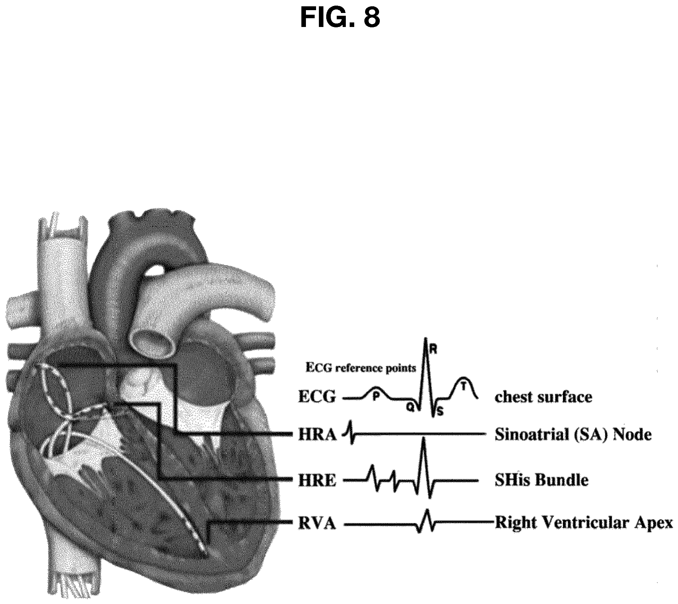

FIG. 7 is a perspective view showing a detection of the His bundle with the capture catheter according to the preferred exemplary embodiment of the present invention. FIG. 7a shows a case where one electrocardiogram sensor is provided, and FIG. 7b shows a case where a plurality of electrocardiogram sensors is provided. FIG. 8 is a diagram showing an electrocardiogram waveform when the electrocardiogram sensor is in touch with a part of the His bundle.

Referring to FIGS. 7 and 8, when the electrocardiogram sensor 68 detects the position of the His bundle, an electrocardiogram waveform detected only in the His bundle is generated. The position of the His bundle may be verified by the radiopaque mark 69 provided at an upper part of the electrocardiogram sensor 68. In the case where the plurality of electrocardiogram sensors 68 is provided as shown in FIG. 7b, the position of the His bundle may be identified by giving a number to each of the electrocardiogram sensors 68, and then by verifying the position of the electrocardiogram sensor 68 having the number indicating the detection of the His bundle.

The next step is a step S30 in which the surgical wire is positioned in the right ventricle after having passed through the His bundle. The surgical wire 10 is positioned in the right ventricle after having passed through the His bundle identified in the previous step S20. The surgical wire 10 has a pointed end, passes through the His bundle, and is positioned in the right ventricle. However, in the case where penetrating the His bundle with only the surgical wire 10 is difficult, a perforating catheter 30 is required.

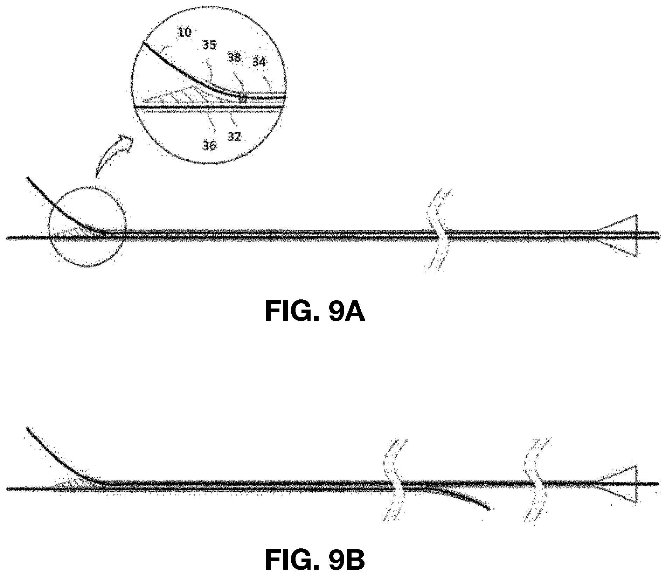

FIG. 9 is a schematic cross-sectional view of a perforating catheter according to the preferred exemplary embodiment of the present invention. FIG. 9a shows a case where the lengths of the first tube and the second tube are the same, and FIG. 9b shows a case where the lengths of the first tube and the second tube are different from each other. Referring to FIG. 9, the perforating catheter 30 having two lumens therein includes: a first tube 32 into which the supporting wire 36 is inserted; a second tube 34 into which the surgical wire 10 is inserted, wherein the second tube 34 includes a second tube radiopaque mark 38, and an inclined guide part 35 is provided at the end of the second tube 34. The first tube 32 and the second tube 34 may be made by two tubes in close contact with each other, or may be made by dividing into two spaces by membranes provided inside of a single tube, and the lengths of the two tubes may be the same or may be different from each other.

After the perforating catheter 30 is inserted into the coronary sinus, the supporting wire 36 is inserted into the first tube 32 to play a supportive role when the surgical wire 10 performs the perforation. The surgical wire 10 is inserted into the second tube 34 to perforate the His bundle, and then the surgical wire 10 is positioned in the right ventricle.

Next, a step S40 is performed so that the capture catheter captures the surgical wire, and then guides the surgical wire into the inferior vena cava.

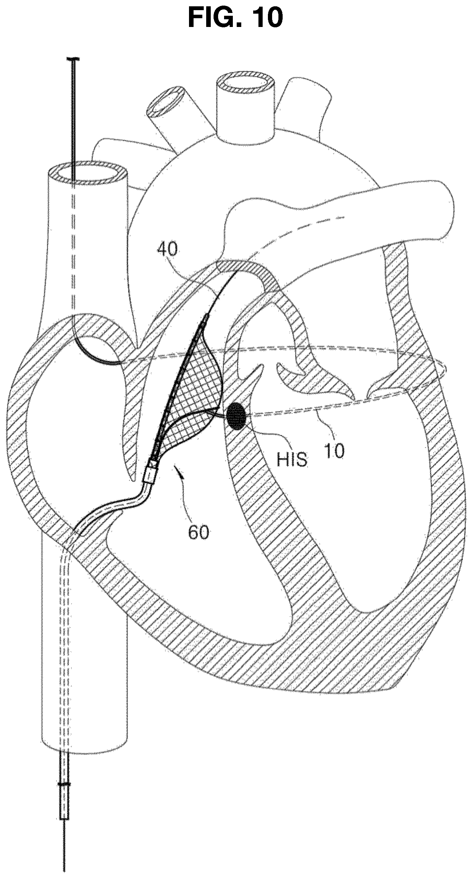

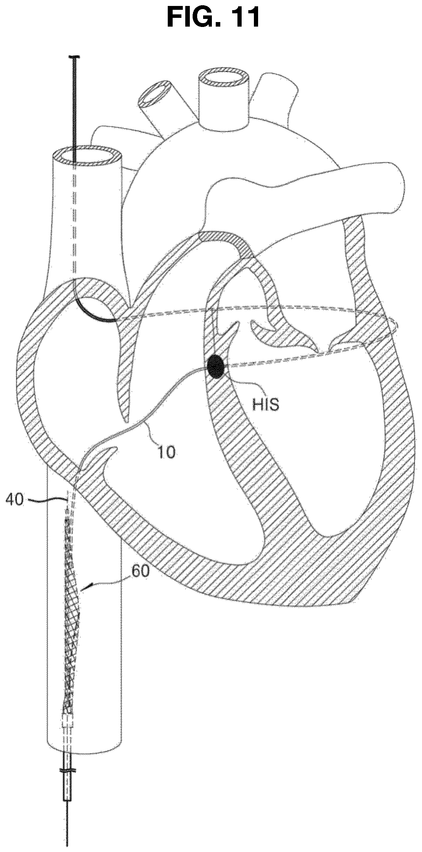

FIG. 10 is a perspective cross-sectional view showing an insertion of a surgical wire, having passed through the His bundle, into a mesh net of the unfolded capture catheter according to the preferred exemplary embodiment of the present invention, and FIG. 11 is a perspective cross-sectional view showing the capture catheter capturing the surgical wire having passed through the His bundle and guiding the surgical wire to the inferior vena cava, according to the preferred exemplary embodiment of the present invention.

Referring to FIGS. 10 and 11, the surgical wire 10 is positioned in the right ventricle after having passed through the His bundle, and the capture catheter 60, having the mesh net 66 unfolded in order to detect the His bundle, is positioned in the right ventricle nearby the His bundle. The surgical wire 10, having passed through the His bundle, passes through the mesh net 66. When the external catheter 64 is pulled downward from the outside, the mesh net 66 is folded, and the surgical wire 10 is captured by the capture catheter 60 and moved together with the capture catheter 60. The capture catheter 60 with the surgical wire 10 captured therein may be guided into the tricuspid valve and the inferior vena cava in sequence, and may be taken out of the patient's body. Accordingly, one end of the surgical wire 10 is positioned outside the upper part of the patient's body, and the other end thereof, taken out to the outside of the patient's body through the superior vena cava, the coronary sinus, the septal vein, the His bundle, the right ventricle, the tricuspid valve, and inferior vena cava in sequence, is positioned at the outside of the lower part of the patient's body.

Finally, a step S50 is performed so that the pacemaker lead is inserted along the surgical wire to place the pacemaker lead tip at a position in the His bundle. The pacemaker lead 70 has a lumen formed therein so that the surgical wire 10 may be inserted therein, and the tip of the pacemaker lead 70 may have a pointed shape to easily penetrate the ventricular septum. While the operator grasps and fixes both ends of the surgical wire 10 from outside, the pacemaker lead 70 is inserted along the surgical wire 10 through the superior vena cava, the coronary sinus, and the septal vein in sequence to be positioned in the His bundle.

While holding both ends of the surgical wire, the operator inserts the pacemaker lead, thereby securing sufficient supporting force to safely insert the lead tip of the pacemaker into the tissue of the ventricular septum.

According to the present invention as described above, the position of the His bundle may be easily detected by coupling the electrocardiogram sensor 68 to the capture catheter 60, and the electrical stimulation may be effectively transmitted by inserting the tip of the pacemaker lead 70 into the His bundle. In addition, since the surgical wire 10 serves as a support, the pacemaker lead 70 may be stably and easily inserted into the His bundle.

Although the exemplary embodiments of the present invention have been described above with reference to the accompanying drawings, it will be understood that those skilled in the art to which the present invention pertains may implement the present invention in other specific forms without departing from the technical scope or essential features thereof. Therefore, the exemplary embodiments described above are to be understood in all respects as illustrative and not restrictive.

## Claims

1. A device for placing, in a His bundle, a pacemaker lead (70) tip having passed through the coronary sinus, the device comprising:
a surgical wire (10);
a capture catheter (60) configured to capture the surgical wire (10) positioned in the right ventricle; and
a pacemaker lead (70) having a lumen formed therein;
**characterized in that**
the capture catheter (60) has an electrocardiogram sensor (68) installed therein configured to identify a position of the His bundle in the ventricular septum and the surgical wire (10) is configured to be inserted into the lumen of the pacemaker lead (70) so that the pacemaker lead (70) tip is insertable into the His bundle along the surgical wire (10).

2. The device of claim 1, wherein the capture catheter (60) comprises: a central catheter (62) having a lumen formed therein; an external catheter (64) having the central catheter (62) inserted therein, the central catheter (62) being movable upward and downward; a mesh net (66) having an upper part thereof fixed to the central catheter (62) in a folded state and a lower part thereof fixed to the external catheter (64) in a folded state; and the electrocardiogram sensor (68) coupled to the mesh net (66) and detecting the position of the His bundle so as to verify the position of the His bundle in the ventricular septum.

3. The device of claim 2, wherein, in the electrocardiogram sensor (68), a plurality of electrocardiogram sensors is capable of being coupled to the mesh net (66), whereby the plurality of electrocardiogram sensors is coupled to each different position.

4. The device of claim 2 or 3, wherein the capture catheter (60) is provided with one or more binders (67) binding the mesh net (66) and the central catheter (62), and the binders (67) move upward and downward along the central catheter (62) as the mesh net (66) is unfolded and folded so that the mesh net (66) has a D shape when being unfolded.

5. The device of any one of claims 2 to 4, wherein a radiopaque mark (69) is provided at a position on a surface of the electrocardiogram sensor (68) to be visually identifiable by an imaging device.

6. The device of any one of claims 1 to 5, wherein the pacemaker lead (70) tip has a pointed shape to easily penetrate the ventricular septum.

7. The device of claim 1, wherein the electrocardiogram sensor (68) is attached to a side of the capture catheter (60) and configured to detect a change in the electrocardiogram to verify a position of the His bundle in the ventricular septum.

8. The device of claim 7, wherein the capture catheter (60) comprises: a central catheter (62) having a lumen formed therein; an external catheter (64) having the central catheter (62) inserted therein, the central catheter (62) being movable upward and downward; a mesh net (66) having an upper part thereof fixed to the central catheter (62) in a folded state and a lower part thereof fixed to the external catheter (64) in a folded state; and the electrocardiogram sensor (68) coupled to the mesh net (66) and detecting the position of the His bundle so as to verify the position of the His bundle in the ventricular septum.

9. The device of claim 8, wherein the capture catheter (60) is provided with one or more binders (67) binding the mesh net (66) and the central catheter (62), and the binders (67) move upward and downward along the central catheter (62) as the mesh net (66) is unfolded and folded so that the mesh net (66) has a D shape when being unfolded.

10. The device of claim 8 or 9, wherein, in the electrocardiogram sensor (68), a plurality of electrocardiogram sensors is capable of being coupled to the mesh net (66), whereby the plurality of electrocardiogram sensors is coupled to each different position.

11. The device of any one of claims 8 to 10, wherein a radiopaque mark (69) is provided at a position on a surface of the electrocardiogram sensor (68) to be visually identifiable by an imaging device.

## Patentansprüche

1. Vorrichtung zum Platzieren der Spitze eines Schrittmacheranschlusses (70), der durch den Koronarsinus geführt wurde, in einem His-Bündel, wobei die Vorrichtung umfasst:
einen chirurgischen Draht (10);
einen Fangkatheter (60), der so konfiguriert ist, dass er den chirurgischen Draht (10), der sich in der rechten Kammer befindet, auffangen kann; und
einen Schrittmacheranschluss (70), der ein darin gebildetes Lumen aufweist;
**dadurch gekennzeichnet, dass**
der Fangkatheter (60) einen darin montierten Elektrokardiogrammsensor (68) aufweist, der so konfiguriert ist, dass er die Position des His-Bündels in der Kammerscheidewand identifizieren kann, und
der chirurgische Draht (10) so konfiguriert ist, dass er in das Lumen des Schrittmacheranschlusses (70) eingeführt werden kann, so dass die Spitze des Schrittmacheranschlusses (70) entlang des chirurgischen Drahts (10) in das His-Bündel einführbar ist.

2. Vorrichtung gemäß Anspruch 1, wobei der Fangkatheter (60) Folgendes umfasst: einen zentralen Katheter (62), der ein darin gebildetes Lumen aufweist; einen externen Katheter (64), in den der zentrale Katheter (62) eingeführt ist, wobei der zentrale Katheter (62) nach oben und unten beweglich ist; ein Netz (66), das einen oberen Teil aufweist, der in einem gefalteten Zustand am zentralen Katheter (62) fixiert ist, und einen unteren Teil aufweist, der in einem gefalteten Zustand am externen Katheter (64) fixiert ist; und der Elektrokardiogrammsensor (68) an das Netz (66) gekoppelt ist und die Position des His-Bündels nachweist, um die Position des His-Bündels in der Kammerscheidewand zu überprüfen.

3. Vorrichtung gemäß Anspruch 2, wobei in dem Elektrokardiogrammsensor (68) eine Vielzahl von Elektrokardiogrammsensoren an das Netz (66) gekoppelt werden können, wodurch die Vielzahl von Elektrokardiogrammsensoren jeweils an unterschiedliche Positionen gekoppelt sind.

4. Vorrichtung gemäß Anspruch 2 oder 3, wobei der Fangkatheter (60) mit einem oder mehreren Bindern (67) versehen ist, die das Netz (66) und den zentralen Katheter (62) aneinander binden, und sich die Binder (67) entlang des zentralen Katheters (62) nach oben bzw. nach unten bewegen, während das Netz (66) entfaltet bzw. zusammengefaltet wird, so dass das Netz (66) eine D-Form aufweist, wenn es gerade entfaltet wird.

5. Vorrichtung gemäß einem der Ansprüche 2 bis 4, wobei sich ein strahlenundurchlässiger Marker (69) auf einer solchen Position auf einer Oberfläche des Elektrokardiogrammsensors (68) befindet, dass er durch eine Bildgebungsvorrichtung optisch identifizierbar ist.

6. Vorrichtung gemäß einem der Ansprüche 1 bis 5, wobei die Spitze des Schrittmacheranschlusses (70) eine zugespitzte Form hat, so dass sie leicht in die Kammerscheidewand eindringen kann.

7. Vorrichtung gemäß Anspruch 1, wobei der Elektrokardiogrammsensor (68) an einer Seite des Fangkatheters (60) befestigt ist und so konfiguriert ist, dass er eine Änderung im Elektrokardiogramm nachweisen kann, um die Position des His-Bündels in der Kammerscheidewand zu überprüfen.

8. Vorrichtung gemäß Anspruch 7, wobei der Fangkatheter (60) Folgendes umfasst: einen zentralen Katheter (62), der ein darin gebildetes Lumen aufweist; einen externen Katheter (64), in den der zentrale Katheter (62) eingeführt ist, wobei der zentrale Katheter (62) nach oben und unten beweglich ist; ein Netz (66), das einen oberen Teil aufweist, der in einem gefalteten Zustand am zentralen Katheter (62) fixiert ist, und einen unteren Teil aufweist, der in einem gefalteten Zustand am externen Katheter (64) fixiert ist; und der Elektrokardiogrammsensor (68) an das Netz (66) gekoppelt ist und die Position des His-Bündels nachweist, um die Position des His-Bündels in der Kammerscheidewand zu überprüfen.

9. Vorrichtung gemäß Anspruch 8, wobei der Fangkatheter (60) mit einem oder mehreren Bindern (67) versehen ist, die das Netz (66) und den zentralen Katheter (62) aneinander binden, und sich die Binder (67) entlang des zentralen Katheters (62) nach oben bzw. nach unten bewegen, während das Netz (66) entfaltet bzw. zusammengefaltet wird, so dass das Netz (66) eine D-Form aufweist, wenn es gerade entfaltet wird.

10. Vorrichtung gemäß Anspruch 8 oder 9, wobei in dem Elektrokardiogrammsensor (68) eine Vielzahl von Elektrokardiogrammsensoren an das Netz (66) gekoppelt werden können, wodurch die Vielzahl von Elektrokardiogrammsensoren jeweils an unterschiedliche Positionen gekoppelt sind.

11. Vorrichtung gemäß einem der Ansprüche 8 bis 10, wobei sich ein strahlenundurchlässiger Marker (69) auf einer solchen Position auf einer Oberfläche des Elektrokardiogrammsensors (68) befindet, dass er durch eine Bildgebungsvorrichtung optisch identifizierbar ist.

## Revendications

1. Dispositif de mise en place, dans un faisceau de His, d'une pointe d'une sonde de stimulateur cardiaque (70) ayant traversé le sinus coronaire, le dispositif comprenant :
un fil chirurgical (10) ;
un cathéter de capture (60) configuré pour capturer le fil chirurgical (10) positionné dans le ventricule droit ; et
une sonde de stimulateur cardiaque (70) ayant une lumière formée en son sein ;
**caractérisé en ce que**
le cathéter de capture (60) a un capteur d'électrocardiogramme (68) installé en son sein configuré pour identifier une position du faisceau de His dans le septum interventriculaire et
le fil chirurgical (10) est configuré pour être inséré dans la lumière de la sonde de stimulateur cardiaque (70) de sorte que la pointe de la sonde de stimulateur cardiaque (70) peut être insérée dans le faisceau de His le long du fil chirurgical (10).

2. Dispositif selon la revendication 1, dans lequel le cathéter de capture (60) comprend : un cathéter central (62) ayant une lumière formée en son sein ; un cathéter externe (64) ayant le cathéter central (62) inséré en son sein, le cathéter central (62) étant mobile vers le haut et vers le bas ; un filet à mailles (66) dont une partie supérieure est fixée au cathéter central (62) dans un état plié et dont une partie inférieure est fixée au cathéter externe (64) dans un état plié ; et le capteur d'électrocardiogramme (68) couplé au filet à mailles (66) et détectant la position du faisceau de His de façon à vérifier la position du faisceau de His dans le septum interventriculaire.

3. Dispositif selon la revendication 2, dans lequel, dans le capteur d'électrocardiogramme (68), une pluralité de capteurs d'électrocardiogramme est capable d'être couplée au filet à mailles (66), moyennant quoi la pluralité de capteurs d'électrocardiogramme est couplée à chaque position différente.

4. Dispositif selon la revendication 2 ou 3, dans lequel le cathéter de capture (60) est pourvu d'un ou de plusieurs liens (67) liant le filet à mailles (66) et le cathéter central (62), et les liens (67) se déplacent vers le haut et vers le bas le long du cathéter central (62) lorsque le filet à mailles (66) est déplié et plié de sorte que le filet à mailles (66) a une forme de D lorsqu'il est déplié.

5. Dispositif selon l'une quelconque des revendications 2 à 4, dans lequel une marque radio-opaque (69) est prévue à une position sur une surface du capteur d'électrocardiogramme (68) pour être visuellement identifiable par un dispositif d'imagerie.

6. Dispositif selon l'une quelconque des revendications 1 à 5, dans lequel la pointe de la sonde de stimulateur cardiaque (70) a une forme pointue pour pénétrer facilement dans le septum interventriculaire.

7. Dispositif selon la revendication 1, dans lequel le capteur d'électrocardiogramme (68) est attaché à un côté du cathéter de capture (60) et configuré pour détecter un changement dans l'électrocardiogramme afin de vérifier une position du faisceau de His dans le septum interventriculaire.

8. Dispositif selon la revendication 7, dans lequel le cathéter de capture (60) comprend : un cathéter central (62) ayant une lumière formée en son sein ; un cathéter externe (64) ayant le cathéter central (62) inséré en son sein, le cathéter central (62) étant mobile vers le haut et vers le bas ; un filet à mailles (66) dont une partie supérieure est fixée au cathéter central (62) dans un état plié et dont une partie inférieure est fixée au cathéter externe (64) dans un état plié ; et le capteur d'électrocardiogramme (68) couplé au filet à mailles (66) et détectant la position du faisceau de His de façon à vérifier la position du faisceau de His dans le septum interventriculaire.

9. Dispositif selon la revendication 8, dans lequel le cathéter de capture (60) est pourvu d'un ou de plusieurs liens (67) liant le filet à mailles (66) et le cathéter central (62), et les liens (67) se déplacent vers le haut et vers le bas le long du cathéter central (62) lorsque le filet à mailles (66) est déplié et plié de sorte que le filet à mailles (66) a une forme de D lorsqu'il est déplié.

10. Dispositif selon la revendication 8 ou 9, dans lequel, dans le capteur d'électrocardiogramme (68), une pluralité de capteurs d'électrocardiogramme est capable d'être couplée au filet à mailles (66), moyennant quoi la pluralité de capteurs d'électrocardiogramme est couplée à chaque position différente.

11. Dispositif selon l'une quelconque des revendications 8 à 10,
dans lequel une marque radio-opaque (69) est prévue à une position sur une surface du capteur d'électrocardiogramme (68) pour être visuellement identifiable par un dispositif d'imagerie.
